# EUROPEAN PATENT SPECIFICATION

(11) **EP 3 512 827 B1**
(45) Date of publication and mention of the grant of the patent: **08.04.2020**
(21) Application number: 17798334.3
(22) Date of filing: 20.10.2017
(51) Int. Cl.: C07C 45/73, C07C 49/04

(54) **PROCESS FOR THE PRODUCTION OF METHYL ISOBUTYL KETONE FROM ACETONE**
VERFAHREN ZUR HERSTELLUNG VON METHYLISOBUTYLKETON AUS ACETON
PROCÉDÉ DE PRODUCTION DE MÉTHYLISOBUTYLCÉTONE À PARTIR D'ACÉTONE

(30) Priority: 21.10.2016 IT 201600106503
(43) Date of publication of application: 24.07.2019
(73) Proprietor: Conser SPA, 00144 Roma (IT)
(72) Inventor: VISIGALLI, Renzo, 00164 Roma (IT)
(74) Representative: Sarpi, Maurizio
(86) International application number: PCT/IB2017/056539
(87) International publication number: WO 2018/073803

(56) References cited:
- CN-A- 105 237 373
- US-A1- 2009 253 940
- US-A1- 2010 222 612

## Description

### Field of application of the invention

The present invention relates to a process for the production of methyl isobutyl ketone (MIBK) from acetone or dimethyl ketone (DMK), in particular it relates to a one-step hydrogenation of acetone process producing methyl isobutyl ketone.

### Prior art

Methyl isobutyl ketone can be produced from acetone or iso-propyl alcohol (IPA) according to the following processes:
a. from dimethyl ketone in a three stages process via dimethyl ketone aldol condensation to diacetone alcohol (DAA), followed by diacetone alcohol dehydration to mesityl oxide (MSO) and mesityl oxide hydrogenation to methyl isobutyl ketone;
b. in a single stage process by reaction of dimethyl ketone with hydrogen;
c. as a coproduct in iso-propyl alcohol dehydrogenation to dimethyl ketone.

Actually processes (a) and (b) are the most applied in the industry.

The three-steps process is that originally used the for methyl isobutyl ketone production: at first dimethyl ketone undergoes aldol condensation in presence of an alkali catalyst to form diacetone alcohol. Then diacetone alcohol is selectively dehydrated to mesityl oxide in liquid phase at 90-130°C in presence of an acid catalyst, such as sulfuric acid or phosphoric acid. Finally, mesityl oxide carbon double bond is selectively hydrogenated to methyl isobutyl ketone in liquid-gas phase in presence of various metal catalyst, palladium (Pd) is generally preferred.

Methyl isobutyl ketone aldol condensation, dehydration and hydrogenation may be combined in a single process step: reactions occur in presence of a suited catalyst. The one-step process is presently considered superior compared to the three-steps process as it presents lower investment cost, less expensive construction materials and friendly environmental process conditions.

In the one-step process methyl isobutyl ketone is produced by reacting dimethyl ketone with hydrogen in presence of a suitable catalyst in a temperature range from 100 to 140°C and at a pressure from 5 to 50 barg. The reaction is highly exothermic and generally occurs in vertical isothermal tubular reactor type with tubes filled in with the catalyst. Two reactors in parallel are usually provided for operating flexibility during catalyst regeneration phase.

In order to control reaction temperature increase due to the exothermic reaction adiabatic packed type reactor with external circulation and cooling of the reaction mixture may also be used.

The catalyst predominantly used in industrial applications consists of a cationic resin containing a noble metal, preferably palladium, in mono-atomic form distribution, such as, Lewatit® manufactured by Lanxess Deutschland GmbH, Amberlyst manufactured by Rohm and Haas Company, USA, whose properties and characteristics are well known since 1976.

U.S. Pat. 7,767,863 describes an improved method for the manufacture of methyl isobutyl ketone and diisobutyl ketone from dimethyl ketone and/or iso-propyl alcohol, by reacting, in the presence of an aldol condensation catalyst, a gaseous mixture comprising hydrogen and dimethyl ketone and/or iso-propyl alcohol. The method enables the use of a reaction pressure greater than 207 kPa (30 psig) to increase the ratio of methyl isobutyl ketone to diisobutyl ketone and the manufacture of methyl isobutyl ketone and diisobutyl ketone by reacting, in the presence of an aldol condensation catalyst, a gaseous mixture consisting essentially of dimethyl ketone and/or IPA and optionally water.

U.S. Pat. 8,101,805 discloses a low-pressure one-step gas-phase process for the production and recovery of methyl isobutyl ketone. The process is designed to recover the additional heat associated with the reactor effluent via heating acetone feed and recycle before entering the reactor, a compressor is introduced to the gas-phase process to increase slightly the reactor effluent pressure before this effluent is cooled and fed to a flash drum.

U.S. Pat. 6,008,416 discloses a process for the production of methyl isobutyl ketone from acetone and hydrogen utilizing a catalytic distillation column reactor, wherein reactions take place in a reaction zone with the reaction products being removed from the reaction zone and unreacted acetone being refluxed, the equilibrium is thus continuously disturbed allowing for greater than equilibrium conversion of acetone.

U.S. Pat. 6,518,462 and U.S. Pat. 6,762,328 describe a process for producing methyl isobutyl ketone including dimethyl ketone into a catalytic reactive distillation column where some of dimethyl ketone is converted to MSO, water and, optionally, diacetone alcohol and/or other by products. Product stream containing mesityl oxide and hydrogen are fed into a reaction zone wherein mesityl oxide present in the product stream and hydrogen react to form methyl isobutyl ketone. A methyl isobutyl ketone rich product stream is withdrawn from this reaction zone.

Single step processes using a Pd doped catalyst are described in, for example, U.S. Pat. 3,574,763; U.S. Pat. 3,953,517, U.S. Pat. 5,684,207, South African complete patent application No. 2004/8988 and U.S. Pat. 2009/0253940.

For the process described in U.S. Pat. 3,953,517 by using an adiabatic type reactor a 34.4% dimethyl ketone conversion is reported having a 96.5%. selectivity to methyl isobutyl ketone. However, said selectivity is obtained by carrying out the reaction at a pressure range higher than 60 bar with negative impact on investment cost in case of industrial application.

In U.S. patent application 2009/0253940 a dimethyl ketone conversion of 29.5% and a selectivity to methyl isobutyl ketone of 87.3% by a tubular reactor operating at 30 bar and a temperature of 110-130°C.

U.S. Patent 5,684,207 discloses a process for the production of methyl isobutyl ketone with increased yield of methyl isobutyl ketone and lifetime of the catalyst by using an organic acid to modify the cation exchange resin catalyst. Dimethyl ketone conversions slightly above 40% with a selectivity of about 90-91% to methyl isobutyl ketone by using the described catalyst in a fixed bed type reactor are claimed.

The Chinese document CN-A-105 237 373 discloses the process for the production of methyl isobutyl ketone by the hydrogenation of acetone in two reactors also arranged in series wherein acetone is fed into the first continuous reactor, hydrogen is fed into the first continuous reactor, and from the first reactor to the second reactor, liquid product is fed from the first reactor to the second one, the liquid product is taken from the second reactor for separation.

However, these known solutions, while achieving high conversion and selectivity, have pressures and operating temperatures that provide considerable plant and operating costs.

### Summary of the Invention

The object of the present invention is to provide a one-step hydrogenation of acetone process for the production of methyl isobutyl ketone, and the relative production plant, optimized and having a conversion efficiency of dimethyl ketone in methyl isobutyl ketone from acetone higher of 36% with a methyl isobutyl ketone selectivity greater than 94% and operating at reduced pressures and temperatures. These features allow to reduce costs and to overcome the limits that still affect the current production of methyl isobutyl ketone from acetone with respect to the prior art.

For a better understanding of the present invention, two embodiments are now described, purely by way of non-limiting example, with reference to the accompanying drawings.

### Description of the figures

Figure 1 shows a diagram of a first embodiment according to the invention of a production plant of methyl isobutyl ketone from acetone.
Figure 2 shows a scheme of a second embodiment according to the invention of a production plant of methyl isobutyl ketone from acetone.

### Detailed description of the invention

The process for producing methyl isobutyl ketone from acetone involves the use of raw materials such as dimethyl ketone and hydrogen. In addition, the process for the production of dimethyl isobutyl ketone from acetone comprises the step of performing a hydrogenation reaction of dimethyl ketone in two different reactors in series forming the reaction section downstream of which a recovery section is provided.

The process for producing methyl isobutyl ketone from acetone according to the invention comprises the following steps:
- feeding to a first continuous reactor a stream of hydrogen and a current consisting of fresh dimethyl ketone, unconverted dimethyl ketone recirculated by a recovery section and a liquid stream effluent from a second reactor in series with the first reactor;
- feeding to a second continuous reactor a gas stream rich in hydrogen and effluent from the first reactor, and a liquid stream consisting of fresh dimethyl ketone and a fraction between 25% and 100% of the unconverted dimethyl ketone recirculated from the recovery section;

- feeding the liquid phase produced in the first reactor to the dimethyl ketone recovery section;
- separating the unconverted dimethyl ketone, to be recycled to the first and second reactors, the methyl isobutyl ketone produced in the first and second reactors, by-products having boiling point lower than methyl isobutyl ketone and water from the current fed to the recovery section.

More particularly, the process according to the invention comprises feeding fresh dimethyl ketone and unconverted and recycled dimethyl ketone from the recovery section 110 to a first reactor 101 and to a second reactor 102, in series.

A pure hydrogen stream is fed to the first reactor 101. The gaseous stream leaving the first reactor 101 is fed to the second reactor 102.

According to a process embodiment, the process comprises a filtering step into a filter 104a of the liquid stream produced in the second reactor 102 and a phase of flow of said filtered liquid stream to the first reactor 101 by means of a pump 106a along with part of the recycled dimethyl ketone from the recovery section 110.

Furthermore, the process involves exhaling excess hydrogen from the second reactor 102 allowing an increase in partial hydrogen pressure within the second reactor 102 and, thereby, favoring the hydrogenation reaction of mesityl oxide to methyl isobutyl ketone. The process comprises obtaining a liquid stream comprising the produced methyl isobutyl ketone, unconverted dimethyl ketone and the reaction by-products and outputing said liquid stream to the recovery section 110, wherein the unconverted dimethyl ketone is separated, which is then recycled to the reaction section, and fed for a fraction between 25 and 100% to the second reactor 102 and for the remaining fraction between 75% and 0% to the first reactor 101. Advantageously, the stream of raw methyl isobutyl ketone is purified to obtain pure methyl isobutyl ketone. By-products having a lower boiling point of methyl isobutyl ketone and water are in turn separated into the separation section.

The excess hydrogen leaving the first reactor 101 is fed to the second reactor 102.

According to one aspect of the invention, the method comprises the steps of:
- cooling a gaseous stream effluent from the second reactor;
- supplying said gaseous stream to an eductor, and
- recirculating said gaseous stream to said second reactor.

As mentioned above, the present invention also relates to a methyl isobutyl ketone from acetone production plant.

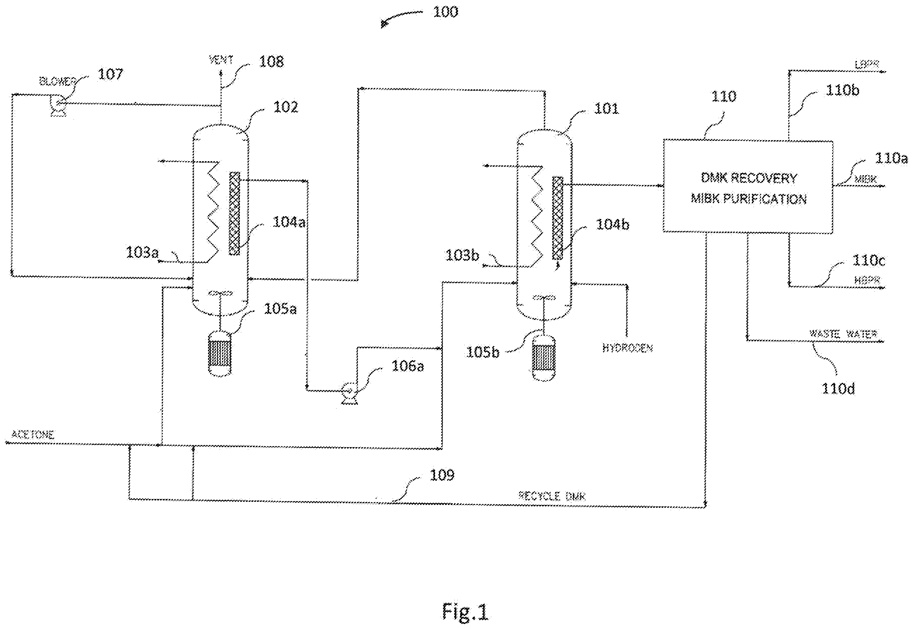

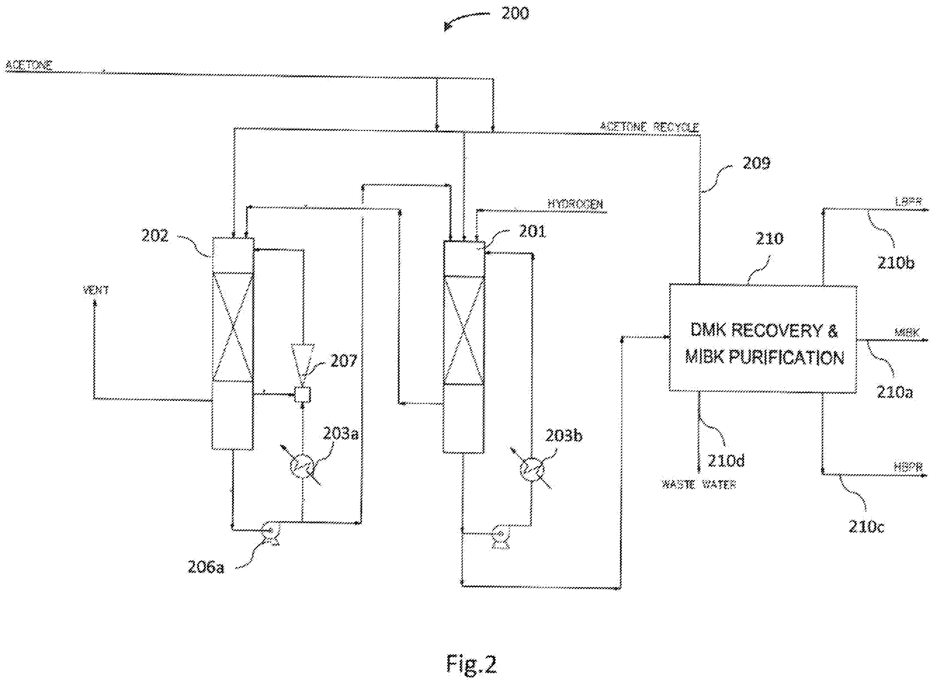

According to a first embodiment, shown in FIG. 1, and a second embodiment, shown in FIG. 2, the plant 100 or 200 comprises a first continuous reactor 101, 201 and a second continuous reactor 102, 202 arranged in series each other; and a recovery section 110, 210 of unconverted dimethyl ketone and purification of the produced methyl isobutyl ketone.

Hydrogen is fed to the first reactor 101, 201, fresh dimethyl ketone and any remaining portion of unconverted dimethyl ketone recirculated from the recovery section 110, 210.

The gaseous stream leaving the first reactor 101, 201 is rich in hydrogen and is fed to the second reactor 102, 202.

In all the embodiments of the invention, 100% of fresh hydrogen is fed to the first reactor 101, 201.

The liquid stream obtained in the first reactor 101, 201 is composed of produced methyl isobutyl ketone, unconverted dimethyl ketone, and reaction by products. Such liquid stream leaving the first reactor 101, 201 is pumped to the recovery section 110, 210 dimethyl ketone and purification of methyl isobutyl ketone, wherein the unconverted dimethyl ketone is separated from the residual compounds to be recycled in part to the second reactor 102, 202 and for the remaining part to the first reactor 101, 201 through a line 109, 209. In particular, the fraction of the unconverted dimethyl ketone recirculated from the recovery section 110, 210 and fed to the second reactor 102, 202 is between 25 and 100% of the total recycle flow rate, and the remaining amount is fed to the first reactor 101, 201. The raw methyl isobutyl ketone stream produced in the first reactor 101, 201 is purified in the recovery section 110, 210 of dimethyl ketone and purification of methyl isobutyl ketone, to obtain pure methyl isobutyl ketone extracted through a line 110a and 210a. The lower and higher boiling point by-products are respectively extracted through lines 110b, 210b and 110c 210c, while the reaction water is discharged through line 110d, 210d.

The excess hydrogen from the first reactor 101, 201 is extracted and forwarded to the second reactor input 102, 202.

In particular, Figure 1 shows a first embodiment of the production plant 100 of isobutyl ketone from acetone, according to which the second reactor 102 for the production of methyl isobutyl ketone is a three fase reactor filled with a solid catalyst, and provided with an inner coil 103a, a filter 104a and a stirrer 105a. Furthermore, the first reactor 101 is a three-phase reactor filled with a solid catalyst and equipped with an internal cooling coil 103b, a filter 104b and a stirrer 105b.

According to one aspect of the invention, the second reactor 102 is provided with a pump 106a and a compressor 107 respectively on the outgoing liquid stream and on the outgoing gas stream and recirculating to the reactor itself.

Advantageously, agitator 105a, 105b enables an adequate mixing between the liquid-solid-gas phase in the first reactor 101 and in the second reactor 102 and the internal temperature is maintained substantially constant by circulating a refrigerant fluid within the serpentins 103a and 103b.

Advantageously, the compressor 107 is used to recycle a portion of excess hydrogen through the second reactor 102, allowing for a increase in hydrogen partial pressure within the second reactor 102, thus favoring the hydrogenation reaction of mesityl oxide into methyl isobutyl ketone.

The liquid stream obtained by the second reactor 102 is filtered into filter 104a and pumped to the inlet of the first reactor 101 by the pump 106a together with the remaining portion of recycled dimethyl ketone from the recycle section 110 of the dimethyl ketone.

Excess hydrogen is discharged from the second reactor 102 through line 108.

Figure 2 shows a second embodiment of the production plant of methyl isobutyl ketone from acetone according to the invention.

In particular according to a second embodiment 200 of the production plant of methyl isobutyl ketone from dimethylketone, the second reactor 202 is of the fixed bed type containing a catalyst packet, a heat exchanger 203a, an eductor 207, and a pump 206a. Furthermore, the first reactor 201 is of fixed bed type with a catalyst pack and a heat exchanger 203b.

The first reactor 201 and the second reactor 202 are connected in series.

According to an aspect of the invention, the liquid stream from the second reactor 202 is addressed through the pump 206a to the exchanger 203a wherein it undergoes a cooling. The liquid stream is then fed to the converter 207 to enable the compression and recirculation of a steam rich in hydrogen leaving the bottom of the second reactor 202 in inlet of the same second reactor 202.

According to an aspect of the invention, the gaseous stream leaving the second reactor 202 is addressed to the exchanger 203a wherein it undergoes a cooling and is then aspirated by the educator 207 and recirculated to the second reactor 202.

The net liquid stream from the second reactor 202 is pumped through the pump 206a to the inlet of the first reactor 201 where it is properly distributed on the catalyst bed.

The remaining amount of dimethyl ketone, i.e. the fresh dimethyl ketone plus the recycled one from the recovery section 210 of the unconverted dimethyl ketone, is also fed to the inlet of the first reactor 201 together with 100% of the fresh hydrogen.

The liquid stream leaving the first reactor 201 may be cooled or not by means of the exchanger 203b and recycled to the input of the first reactor 201 if necessary to enable the liquid temperature control within the first reactor 201.

The excess hydrogen is discharged from the bottom of the first reactor 201 and sent to the inlet of the second reactor 202.

According to an aspect of the invention, the heat exchanger 203a is used for cooling a gaseous stream outgoing from the second reactor 202 and subsequently the gas stream is aspirated by the educator 207 and recirculated to the second reactor 202.

This configuration has the advantage of reducing the costs associated with the size of the heat exchanger 203a and enables better control of the operating temperature of the second reactor 202 following evaporation of the liquid for saturation of the gaseous phase.

According to an aspect of the invention in the second reactor 102, 202 and in the first reactor 101, 201, the dimethyl ketone hydrogenation reaction is carried out in the presence of a solid palladium-doped acid resin-type catalyst.

According to another aspect of the invention in the second reactor 102, 202 and in the first reactor 101, 201, the hydrogenation reaction of the dimethyl ketone is carried out at a temperature between 100°C and 140°C.

Preferably, according to another aspect of the invention in the second reactor 102, 202 and in the first reactor 101, 201, the hydrogenation reaction of the dimethyl ketone is carried out at a temperature between 110°C and 130°C.

According to a further aspect of the invention in the second reactor 102, 202 and in the first reactor 101, 201, the hydrogenation of the dimethyl ketone is carried out at a pressure between 10 and 50 bargs. Preferably, according to another aspect of the invention in the second reactor 102, 202 and in the first reactor 101, 201, the hydrogenation reaction of the dimethyl ketone is carried out at a pressure of between 20 and 40 bargs.

The applicant has carried out numerous laboratory tests aimed at demonstrating the actual optimization achieved in the production process, the increase in conversion and selectivity in methyl isobutyl ketone. The tests, whose results are herewith reported, were carried out in both isothermal and adiabatic reactors under the following operating conditions:
- pressure in the reactor from 8 to 30 bar;
- the outlet temperature from the reactor between 100°C and 140°C, preferably between 120°C and 130°C;
- Lewatite K2624 and Amberlyst CH28 catalyst.

Buchi type glass reactor with external jacket having a total volume of 1 liter has been used for preliminary batch tests at 8 barg in order to compare the performances of the two catalysts.

Similar tests at higher pressure (30 barg) have been carried out in a Parr type steel reactor having a total volume of 300 ml and internal agitator. Dimethyl ketone conversion and selectivity to methyl isobuti ketone were not substantially different with the two catalysts.

A set of test operating in continuous has been also carried out by using a tubular jacketed reactor (Sotelem type reactor) having an internal diameter of 16 mm and 300 mm length (total volume about 50 cu.cm.) filled in with the catalyst. Dimethyl ketone was fed by a piston pump having a capacity of 20 to 150 ml/h while hydrogen flow rate is controlled by a flow meter in the range from 1 to 12 l/hr.

The reactors effluent composition has been analyzed by a Perkin Elmer Clarus 600 gaschromatograph, including a RTX-PONA column and a flame ionization detector. Calibration standards have been prepared by weighing each component after dissolution in acetone in a typical concentration range as obtained during DMK hydrogenation to methyl isobuti ketone.

The reaction byproducts have been identified by Gas chromatography mass spectrometry analysis. The GC-MS chromatogram and the components as shown in Tab. 1 have been identified.

| Tab. 1 - GC-MS chromatogram of Acetone and reaction products | |
|---|---|
| *Time, min* | *Component* |
| 4.90 | dimethyl ketone |
| 5.12 | iso-propyl alcohol |
| 5.95 | 2-methyl pentane |
| 6.33 | 2 methyl pentene |
| 6.74 | tetra methyl ethylene |
| 9.73 | methyl isobutil ketone |
| 11.38 | mesityl oxide |
| 12.05 | diacetone alcohol |
| 12.51 | 1,1,3 trimethyl cyclohexane |
| 12.77 | 3,3 di-methyl pentene |
| 13.53 | 3,3,5 tri-methyl cycle hexane |
| 15.15 | di-iso butyl ketone |
| 15.24 | mesitylene |
| 15.48 | 4,6 di-methyl 2 heptanone |
| 16.35 | di-hydro isophorone |
| 19.13 | isobutyl 2,5 di-methyl benzene |

### Experimental part

The invention will now be below described with particular reference to some non limitative examples.

### Example 1.

A test has been carried out in a Parr type jacketed reactor made in stainless steel, having a total volume of 300 ml provided with electric heating system and including a temperature control system and a mechanical agitator. Hydrogen flow rate was controlled by a mass flowmeter and operating temperature, pressure and hydrogen rate was registered.

The simulation of the operation of the first reactor was performed by filling 100 gr of dimethyl ketone and 7.2 gr of dry catalyst (Lewatite K2624). Temperature and pressure were gradually increased to preset values of 122 °C and 30 bar by hydrogen injection. Agitation of the liquid mass was maintained at 500 rpm.

A total dimethyl ketone conversion of 30.1% and a selectivity to methyl isobutyl ketone of 96.7% was achieved after 120 minutes of reaction time (see Table 2 test 7).

The simulation of the operation of the second reactor was performed by filling 50 gr of product from the previous test, 50 gr of fresh dimethyl ketone and 7.2 gr of catalyst recovered from the previous test.

The reactor operating temperature and pressure were set at 122 °C and 30 bar by hydrogen injection. After 120 minutes operation a total dimethyl ketone conversion of 36.6% and a total selectivity to methyl isobutyl ketone of 97.5% were achieved (see Table 2 - test 8).

| Tab. 2 - Reactor feed-effluent compositions | | | |
|---|---|---|---|
| | Test 7 | Test 8 | |
| Reactor feed | DMK | 50% test 7 + 50% DMK | |
| Total Feed (gr) | 100 | 100 | |
| Catalyst (gr) | 7.2 | 7.2(*) | |
| Op. temperature (°C) | 122 | 122 | |
| Op. pressure (bar) | 30 | 30 | |
| Reaction Time (min) | 120 | 120 | |

| Component | % out | % in | % out |
|---|---|---|---|
| dimethyl ketone | 69.90 | 80.99 | 63.39 |
| iso-propyl alcohol | 0.07 | 0.04 | 0.09 |
| 2 methylpentane | 0.44 | 0.09 | 0.48 |
| methyl isobutil ketone | 25.10 | 16.13 | 30.76 |
| diacetone alcohol | 0.19 | 0.03 | 0.10 |
| di-isobutyl ketone | 0.32 | 0.20 | 0.50 |
| others | balance | balance | balance |
| water | 3.81 | 2.40 | 4.44 |
| Total Conversion | 30.1 | | 36.6 |
| Total Selectivity | 96.7 | | 97.5 |

| | | | |
|---|---|---|---|
| (*) Catalyst recovered from previous test run. | | | |

### Example 2.

A test has been carried out in the same apparatus as described in previous example 1.

The simulation of the operation of the first reactor was performed by filling 100 gr of dimethyl ketone and 5.0 gr of dry catalyst (Lewatite K2624). Temperature and pressure were gradually increased to preset values of 120 °C and 30 bar by hydrogen injection. Agitation of the liquid mass was maintained at 500 rpm.

A total dimethyl ketone conversion of 30.8% and a selectivity to methyl isobutyl ketone of 94.3% was achieved after 90 minutes of reaction time (see Table 3 test 13).

The simulation of the operation of the second reactor was performed by filling 80 gr of product from the previous test, 20 gr of fresh dimethyl ketone and 5.0 gr of catalyst recovered from the previous test.

The reactor was pressurized by hydrogen injection up to 30 bar and temperature increased to 125 °C. After 90 minutes of operation a total dimethyl ketone conversion of 37.9 % and a total selectivity to methyl isobutyl ketone of 93.6 % were achieved (see Table 3 - test 14).

| Tab. 3 - Reactor feed-effluent compositions | | | |
|---|---|---|---|
| | Test 13 | Test 14 | |
| Reactor feed | DMK | 80% test 13 + 20% DMK | |
| Total Feed (gr) | 100 | 100 | |
| Catalyst (gr) | 5.0 | 5.0(*) | |
| Op. temperature (°C) | 120 | 125 | |
| Op. pressure (bar) | 30 | 30 | |
| Reaction Time (min) | 90 | 90 | |

| Component | % out | % in | % out |
|---|---|---|---|
| dimethyl ketone | 67.71 | 75.57 | 62.11 |
| iso-propyl alcohol | 0.21 | 0.17 | 0.17 |
| 2 methylpentane | 0.32 | 0.31 | 0.39 |
| methyl isobutil ketone | 26.11 | 20.07 | 30.58 |
| diacetone alcohol | 0.16 | 0.11 | 0.12 |
| di-isobutyl ketone | 0.30 | 0.23 | 0.46 |
| others | balance | balance | balance |
| water | 5.06 | 3.45 | 5.92 |
| Total Conversion | 32.3 | | 37.9 |
| Total Selectivity | 93.8 | | 93.6 |

| | | | |
|---|---|---|---|
| (*) Catalyst recovered from previous test run. | | | |

### Results

Example 1 is characterized by an equal distribution of the total DMK between the first and the second reactor and represents a case of optimum performances. In fact, the values achieved, 36.6% of total conversion and 97.5% of total selectivity, are the highest values mentioned in the technical literature. It is to be noted the improvement of 6.5% of the conversion compared with the value obtainable from a process using a single reactor processing 100% of the total DMK.

Example 2, compared with example 1, is characterized by:
a) a different distribution of the total DMK, 80% in the first reactor and 20% in the second reactor;
b) a different range of temperatures between the two reactors;
c) a lower retention time;
d) a lower catalyst load.

Example 2 provides reduced investment necessity. In fact the above variants, if transferred in the design of a commercial plant, would result in savings of investment due to reduced volumes of reactors, reduced catalyst loadings and, moreover, in a simpler design of the second reactor, which would not need an internal or external cooling equipment.

The performances of example 2 would remain on the high side by confirming in front of a still high value of the selectivity, a further improved value of the conversion to a value of 37.9%, 7.2% higher than the value obtainable from a process using a single reactor.

## Claims

1. A process for producing methyl isobutyl ketone from acetone comprising the following steps:
- feeding to a first continuous reactor (101, 201) a stream of hydrogen and a stream consisting of fresh dimethyl ketone, unconverted dimethyl ketone recirculated by a recovery section (110, 210) and a liquid stream effluent from a second reactor (102, 202) in series with the first reactor (101, 201);
- feeding to a second continuous reactor (102, 202) a gas stream rich in hydrogen and effluent from the first reactor (101, 201), and a liquid stream consisting of fresh dimethyl ketone and a fraction of the unconverted dimethyl ketone recirculated from the recovery section (110, 210);
- feeding the liquid phase produced in the first reactor (101, 201) to the dimethyl ketone recovery section (110, 210);
- separating the unconverted dimethyl ketone, to be recycled to the first (101, 201) and second (102, 202) reactors, the methyl isobutyl ketone produced in the first (101, 201) and second (102, 202) reactors, by-products having boiling point lower than methyl isobutyl ketone and water from the current fed to the recovery section (110, 210).

2. The process for producing methyl isobutyl ketone from acetone according to claim 1 comprising a step of filtering the liquid stream produced in the second reactor and a step of pumping this filtered liquid stream to the first reactor together with a portion of the recycled dimethyl ketone effluent from the dimethyl ketone recovery section.

3. The process for producing methyl isobutyl ketone from acetone according to claim 1 comprising:
- cooling a gaseous stream effluent from the second reactor;
- supplying said gaseous stream to an eductor, and
- recirculating said gaseous stream to said second reactor.

4. The process for producing methyl isobutyl ketone from acetone according to claim 1 comprising a step of of discharging the excess hydrogen from the second reactor promoting the hydrogenation of mesityl oxide to methyl isobutyl ketone.

5. The process for producing methyl isobutyl ketone from acetone according to claim 1 comprising a step of separating unconverted dimethyl ketone and recirculating unconverted dimethyl ketone to the first and second reactor, and a step of feeding a fraction fraction of the recycled dimethyl ketone between 25 and 100% to the second reactor and a remaining porzion between 0% and 75% to the first reactor.

6. The process for producing methyl isobutyl ketone from acetone according to claim 1 comprising a raw methyl isobutyl ketone stream purification step to obtain pure methyl isobutyl ketone and a step of separating by-products having boiling point lower or higher than methyl isobutyl ketone, and water.

7. The process for producing methyl isobutyl ketone from acetone according to claim 1 wherein in both the reactors the dimethyl chetone hydrogenation is carried out with a solid catalyst of Pd doped on acidic resin type.

8. The process according to claims 1-7 wherein the reaction is carried out at a pressure ranging from 10 to 50 barg.

9. The process according to claim 8 wherein the reaction is carried out at a pressure ranging from 20 to 40 barg.

10. The process according to claims 1-7 wherein the reaction is carried out at a temperature ranging from 100°C to 140°C.

11. The process according to claim 10 wherein the reaction is carried out at a temperature ranging from 110°C to 130°C.

12. The process for producing methyl isobutyl ketone from acetone according to claims 1-7 providing 100% of fresh hydrogen stream to the first reactor.

13. Use of the production plant (100, 200) for producing methyl isobutyl ketone from acetone comprising a first continuous reactor (101, 201) and a second continuous reactor (102, 202) in series, and a recovery section (110, 210) of unconverted dimethyl ketone also configured to purify the methyl isobutyl ketone product wherein said plant is used in the execution of the process of claim 1.

14. Use of the production plant according to claim 13 in the execution of a process of claim 1 wherein the first reactor (101) and the second reactor (102) for the production of methyl isobutyl ketone are filled with a catalyst in solid phase, and are equipped with and internal coil (103a, 103b), a filter (104a, 104b), a stirrer (105a, 105b).

15. Use of the production plant according to claim 13 in the execution of a process of claim 1 wherein the first reactor (201) and the second reactor (202) are fixed bed reactors with catalyst pack and are equipped with a heat exchanger (203a, 203b) for external temperature control, and in that the second reactor (202) is equipped with an educator (207).

## Patentansprüche

1. Prozess zur Herstellung von Methylisobutylketon aus Aceton, der die folgenden Schritte umfasst:
- Zuführen eines Wasserstoffstroms und eines Stroms, der frisches Dimethylketon, nicht umgesetztes Dimethylketon, das mittels einer Wiedergewinnungseinheit (110, 210) rezyklisiert wurde, und einen flüssigen Abstrom eines zweiten Reaktors enthält, der mit dem ersten Reaktor (101, 201) in Serie geschalten ist, zu einem ersten kontinuierlichen Reaktor (101, 201);
- Zuführen eines Gasstroms, der reich an Wasserstoff ist, und eines Abstroms des ersten Reaktors (101, 201), und eines Flüssigkeitsstroms, der aus frischen Dimethylketon und einem Teil des nicht umgesetzten Dimethylketons, das mittels der Wiedergewinnungseinheit (110, 210) rezyklisiert wurde, zu einem zweiten kontinuierlichen Reaktor (102, 202);
- Zuführen der flüssigen Phase, die im ersten Reaktor (101, 201) hergestellt wird, zu der Wiedergewinnungseinheit (110, 210) des Dimethylketons;
- Abtrennen des nicht umgesetzten Dimethylketons, das in den ersten (101, 201) und zweiten (102, 202) Reaktoren recycelt werden soll, des Methylisobutylketons, das in den ersten (101, 201) und zweiten (102, 202) Reaktoren hergestellt wird, von Nebenprodukten mit einem niedrigeren Siedepunkt als Methylisobutylketon und Wasser, vom Strom, der der Wiedergewinnungseinheit (110, 210) zugeführt wird.

2. Prozess zur Herstellung von Methylisobutylketon aus Aceton gemäß Anspruch 1, der einen Schritt des Filterns des Flüssigkeitsstroms, der im zweiten Reaktor hergestellt wird, und einen Schritt des Pumpens dieses gefilterten Flüssigkeitsstroms in den ersten Reaktor zusammen mit einem Teil des rezyklisierten Dimethylketon-Abstroms der Wiedergewinnungseinheit des Dimethylketons umfasst.

3. Prozess zur Herstellung von Methylisobutylketon aus Aceton nach Anspruch 1, umfassend:
- Kühlen eines gasförmigen Abstroms des zweiten Reaktors;
- Einspeisen dieses gasförmigen Stroms in einen Eduktor, und
- Rezyklisieren dieses gasförmigen Stroms in den zweiten Reaktor.

4. Prozess zur Herstellung von Methylisobutylketon aus Aceton gemäß Anspruch 1, der einen Schritt des Ausspeisens des überschüssigen Wasserstoffs aus dem zweiten Reaktor umfasst, um die Hydrierung von Mesityloxid zu Methylisobutylketon zu fördern.

5. Prozess zur Herstellung von Methylisobutylketon aus Aceton gemäß Anspruch 1, der einen Schritt des Abtrennens des nicht umgesetzten Dimethylketons und Rezyklisierens des nicht umgesetzten Dimethylketons in den ersten und zweiten Reaktor, und einen Schritt des Zuführens einer Teilfraktion des rezyklisierten Dimethylketons zwischen 25 und 100% zu dem zweiten Reaktor und eines verbleibenden Teils zwischen 0% und 75% zu dem ersten Reaktor umfasst.

6. Prozess zur Herstellung von Methylisobutylketon aus Aceton gemäß Anspruch 1, der einen Schritt der Reinigung des rohen Methylisobutylketonstroms, um reines Methylisobutylketon zu erhalten, und einen Schritt des Abtrennens der Nebenprodukte mit einem Siedepunkt niedriger oder höher als Methylisobutylketon und Wasser umfasst.

7. Prozess zur Herstellung von Methylisobutylketon aus Aceton gemäß Anspruch 1, wobei die Hydrierung des Dimethylketons in beiden Reaktoren mit einem festen Katalysator des Typs Pd dotiert auf saurem Harz durchgeführt wird.

8. Prozess gemäß den Ansprüchen 1 - 7, wobei die Reaktion bei einem Druck zwischen 10 und 50 barg durchgeführt wird.

9. Prozess gemäß Anspruch 8, wobei die Reaktion bei einem Druck zwischen 20 und 40 barg durchgeführt wird.

10. Prozess gemäß den Ansprüchen 1 - 7, wobei die Reaktion bei einer Temperatur zwischen 100°C und 140 °C durchgeführt wird.

11. Prozess gemäß Anspruch 10, wobei die Reaktion bei einer Temperatur zwischen 110°C du 130°C durchgeführt wird.

12. Prozess zur Herstellung von Methylisobutylketon aus Aceton gemäß den Ansprüchen 1 - 7, bei dem ein Strom aus 100% frischem Wasserstoff dem ersten Reaktor zugeführt wird.

13. Verwendung der Produktionsanlage (100, 200) zur Herstellung von Methylisobutylketon aus Aceton, die einen ersten kontinuierlichen Reaktor (101, 201) und einen zweiten kontinuierlichen Reaktor (102, 202), die in Serie geschalten sind, und eine Wiedergewinnungseinheit (110, 210) des nicht umgesetzten Dimethylketons, die auch dazu konfiguriert ist, um das Methylisobutylketonprodukt zu reinigen, umfasst, wobei diese Anlage zur Durchführung des Prozesses aus Anspruch 1 verwendet wird.

14. Verwendung der Produktionsanlage gemäß Anspruch 13 zur Durchführung des Prozesses aus Anspruch 1, wobei der erste Reaktor (101) und der zweite Reaktor (102) zur Produktion von Methylisobutylketon mit einem Katalysator in fester Phase gefüllt sind und mit einer inneren Spule (103a, 103b), einem Filter (104a, 104b), einem Rührer (105a, 105b) ausgestattet sind.

15. Verwendung der Produktionsanlage gemäß Anspruch 13 zur Durchführung des Prozesses aus Anspruch 1, wobei der erste Reaktor (201) und der zweite Reaktor (202) Festbettreaktoren mit Katalysatorpackung sind und mit einem Wärmetauscher (203a, 203b) zur externen Temperaturkontrolle ausgestattet sind und wobei der zweite Reaktor (207) mit einem Eduktor ausgestattet ist.

## Revendications

1. Procédé de production de méthylisobutylcétone à partir d'acétone comprenant les étapes suivantes :
- introduction dans un premier réacteur continu (101, 201) d'un flux d'hydrogène et d'un flux constitué de diméthylcétone fraîche, de diméthylcétone non convertie remise en circulation par une section de récupération (110, 210) et d'un effluent de flux liquide d'un second réacteur (102, 202) en série avec le premier réacteur (101, 201) ;
- introduction dans un second réacteur continu (102, 202) d'un flux de gaz riche en hydrogène et en effluent du premier réacteur (101, 201), et d'un flux liquide constitué de diméthylcétone fraîche et d'une fraction de diméthylcétone non convertie remise en circulation de la section de récupération (110, 210) ;
- introduction de la phase liquide produite dans le premier réacteur (101, 201) dans la section de récupération de diméthylcétone (110, 210) ;
- séparation de la diméthylcétone non convertie, à recycler aux premier (101, 201) et second (102, 202) réacteurs, de la méthylisobutylcétone produite dans les premier (101, 201) et second (102, 202) réacteurs, des produits intermédiaires ayant un point d'ébullition inférieur à la méthylisobutylcétone et de l'eau du courant introduit dans la section de récupération (110, 210).

2. Procédé de production de méthylisobutylcétone à partir d'acétone selon la revendication 1 comprenant une étape de filtration du flux liquide produit dans le second réacteur et une étape de pompage de ce flux liquide filtré au premier réacteur conjointement à une partie de l'effluent de diméthylcétone recyclé de la section de récupération de diméthylcétone.

3. Procédé de production de méthylisobutylcétone à partir d'acétone selon la revendication 1 comprenant :
- le refroidissement d'un effluent de flux gazeux du second réacteur ;
- la fourniture dudit flux gazeux à un éjecteur, et
- la remise en circulation dudit flux gazeux au dit second réacteur.

4. Procédé de production de méthylisobutylcétone à partir d'acétone selon la revendication 1 comprenant une étape de décharge de l'excès d'hydrogène du second réacteur favorisant l'hydrogénation de l'oxyde de mésityle en méthylisobutylcétone.

5. Procédé de production de méthylisobutylcétone à partir d'acétone selon la revendication 1 comprenant une étape de séparation de la diméthylcétone non convertie et de remise en circulation de la diméthylcétone non convertie auxdits premier et second réacteurs, et une étape d'introduction d'une fraction de la diméthylcétone recyclée entre 25 et 100 % dans le second réacteur et d'une partie restante entre 0 % et 75 % dans le premier réacteur.

6. Procédé de production de méthylisobutylcétone à partir d'acétone selon la revendication 1 comprenant une étape de purification du flux de méthylisobutylcétone brute pour obtenir de la méthylisobutylcétone pure et une étape de séparation des produits intermédiaires ayant un point d'ébullition inférieur ou supérieur à la méthylisobutylcétone, et de l'eau.

7. Procédé de production de méthylisobutylcétone à partir d'acétone selon la revendication 1 dans lequel dans les deux réacteurs l'hydrogénation de la diméthylcétone est réalisée avec un catalyseur solide de Pd dopé sur un type de résine acide.

8. Procédé selon les revendications 1 à 7 dans lequel la réaction est réalisée à une pression dans la plage de 10 à 50 barg.

9. Procédé selon la revendication 8 dans lequel la réaction est réalisée à une pression dans la plage de 20 à 40 barg.

10. Procédé selon les revendications 1 à 7 dans lequel la réaction est réalisée à une température dans la plage de 100 °C à 140 °C.

11. Procédé selon la revendication 10 dans lequel la réaction est réalisée à une température dans la plage de 110 °C à 130 °C.

12. Procédé de production de méthylisobutylcétone à partir d'acétone selon les revendications 1 à 7 fournissant 100 % de flux d'hydrogène frais au premier réacteur.

13. Utilisation de l'installation de production (100, 200) pour produire la méthylisobutylcétone à partir d'acétone comprenant un premier réacteur continu (101, 201) et un second réacteur continu (102, 202) en série, et une section de récupération (110, 210) de diméthylcétone non convertie également configurée pour purifier le produit de méthylisobutylcétone dans lequel ladite installation est utilisée dans l'exécution du procédé selon la revendication 1.

14. Utilisation de l'installation de production selon la revendication 13 dans l'exécution d'un procédé selon la revendication 1 dans lequel le premier réacteur (101) et le second réacteur (102) pour la production de méthylisobutylcétone sont remplis d'un catalyseur en phase solide, et sont équipés d'une bobine interne (103a, 103b), d'un filtre (104a, 104b), d'un agitateur (105a, 105b).

15. Utilisation de l'installation de production selon la revendication 13 dans l'exécution d'un procédé selon la revendication 1 dans lequel le premier réacteur (201) et le second réacteur (202) sont des réacteurs à lit fixe avec un ensemble catalyseur et sont équipés d'un échangeur thermique (203a, 203b) pour la régulation de la température externe, et en ce que le second réacteur (202) est équipé d'un éjecteur (207).
